Europäisches Patentamt

(19) European Patent Office

Office européen des brevets

(11) Publication number: **0 075 378**
**A1**

(12) **EUROPEAN PATENT APPLICATION**

(21) Application number: **82301914.6**

(22) Date of filing: **14.04.82**

(51) Int. Cl.³: **A 61 F 1/03**

(30) Priority: **18.09.81 US 303455**

(43) Date of publication of application: **30.03.83**
**Bulletin 83/13**

(84) Designated Contracting States: **CH DE FR GB LI SE**

(71) Applicant: **CRUCIBLE INC., P.O. Box 88 Parkway West &
Route 60, Pittsburgh Pennsylvania 15230 (US)**

(72) Inventor: **Chandhok, Vijay K., 115 Woodhaven Drive, Mt.
Lebanon Pennsylvania 15228 (US)**

(74) Representative: **Sheader, Brian N. et al, ERIC POTTER &
CLARKSON 5 Market Way Broad Street, Reading
Berkshire, RG1 2BN (GB)**

(54) **Prosthesis device and method of manufacture.**

(57) A prosthesis device for implanting in the human body and particularly adapted for bonding to a bone of the body; the device is preferably made of an alloy, such as titanium, that is inert to human body fluids. The alloy is in powder form and is consolidated by hot isostatic pressing within a mould (10) to provide the device with a fully dense portion (16) and a second portion that is less than fully dense and comprises a compacted alloy layer (14) surrounding a fully dense insert (12). With the powder of the second portion being less than fully dense it is possible to achieve a desired porous surface which facilitates bonding of this portion of the device by bone growth.

1

## PROSTHESIS DEVICE AND
## METHOD OF MANUFACTURE

This invention relates to prosthesis devices and to a method of manufacturing same.

Metal or alloy prosthesis devices which are implanted in the human body and have a portion thereof attached to a bone of the body are conventionally joined to the bone by reaming a cavity in the bone and cementing a portion of the prosthesis device to be attached to the bone within the cavity. An alternative practice, which is more desirable, is to join the prosthesis device to the bone by means of bone growth around the device. This requires that the portion of the device to be attached to the bone by bone growth be porous. If the portion to be joined to the bone is not sufficiently porous bone growth is inhibited and in many cases prevented to result in a failure of attachment of the device to the bone.

It is known to provide the porous metal surface on the portion of the device to be attached to the bone by high temperature sintering of the metal covering said portion. The high temperatures involved in this practice however are detrimental to the physical properties of the metal and
frequently results in the metal of the device being subject
to failure as by cracking.

It is accordingly an object of the present invention to
provide a prosthesis device having a porous surface portion
thereof adapted to be joined to a bone by bone growth, and
to  a method for manufacturing the same, that does not
require high temperature exposure of the metal to detrimentally
affect its physical properties.

The present invention provides a powder metallurgy
prosthesis device for implanting in a human body and having a
portion thereof for bonding to a bone, said device comprising
a first portion of essentially fully dense alloy and a second
portion including a fully dense insert with an alloy powder
layer bonded thereto with said powder layer being less dense
than said essentially fully dense portion.

The invention also provides a powder metallurgy prosthesis
device for implanting in a human body and having a portion
thereof for bonding to a bone, said device comprising a first
portion of essentially fully dense alloy powder and a second
portion including a fully dense insert with an alloy powder layer
bonded thereto with said powder layer being less dense than
said essentially fully dense portion.

The present invention further provides a powder
metallurgy method for producing a prosthesis device for
implanting in a human body and having a portion thereof for
bonding to a bone, said method comprising producing a mould
having a mould cavity conforming substantially to the
configuration of said prosthesis device, filling a first portion of said mould cavity with alloy powder, positioning in a second portion of said mould a fully dense insert, surrounding said insert with a layer of alloy powder said alloy powder in said first position being finer than said alloy powder in said second portion, heating said mould to an elevated temperature and while at elevated temperature subjecting said mould to isostatic pressure to compact said powder, with the powder of said first portion being compacted to essentially full density and the powder of said second portion being bonded to said insert and compacted to a density less than fully dense.

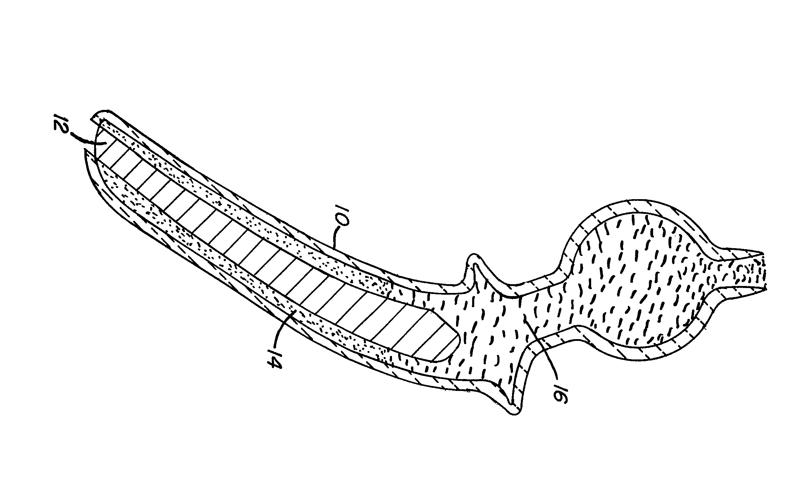

The invention will be more particularly described with reference to the accompanying drawing the single FIGURE of which is a sectional view of one example of a preferred embodiment of the method of the invention.

Broadly, in accordance with the present invention, the prosthesis device is manufactured by hot isostatically compacting powdered metal or alloy from which the device is to be made within a mould having a mould cavity conforming substantially to the final configuration desired in the device. The alloy of the powder should preferably be nonreactive to body fluids to prevent destruction of the device after implanting. Alloys suitable for this purpose include the metal titanium, titanium alloys, cobalt base alloys and stainless steel. The term "alloy" as used herein is intended to cover metals in elemental as well as alloy form. The method of the invention comprises filling the mould with alloy powder with the portion of the mould corresponding to the portion of the prosthesis device to be out of contact with bone growth being of a powder of a density of minus 35 mesh.

The portion of the mould corresponding to the portion of prosthesis device to be connected to the bone by bone growth is provided with a solid insert of metal and surrounded by metal powder of minus 35 plus 60 mesh U.S. Standard. The mould may be of conventional construction for use in hot isostatic compacting such as of a ceramic material. The mould is evacuated and sealed then heated to an elevated temperature of the order of for example 843 to 1871°C (1550 to 3400°F) for hot isostatic compacting in the well known manner by the use of a fluid pressure vessel. The compacting temperature employed will depend upon the specific metal being used, the temperature employed and the duration of pressure application. Typically, with conventional gas pressure vessels, suitable compacting may be achieved at pressures within the range of 704 to 1056 kg/cm$^2$ (10,000 to 15,000 psi). During the compacting operation the temperature and pressure is adjusted to compact the finer powder in the portion of the mould corresponding to the portion of the prosthesis device to be out of contact with bone to full density; whereas, the coarser powder surrounding the solid, fully dense insert when subjected to the same conditions will be compacted to less than full density. Preferably, the denisty will be less than about 70% of full density and specifically have a porosity of about 40%. This will provide by the use of metal particles within the limits minus 35 plus 40 mesh in surface pores in the prosthesis device over the area covered by this powder having pore diameters within the range of 100 to 400 micrometers. This pore size is particularly adapted to facilitating bone growth onto the device and thus achieve bonding between the prosthesis device and the bone.

With respect to the FIGURE in the drawing there is shown in cross section a powder filled ceramic mould 10 having a solid, fully dense metal insert 12 positioned therein and surrounded by metal powder 14 of minus 35 plus 40 mesh. The remainder of the ceramic mould is filled with powder 16 of an identical alloy but being minus 35 mesh and thus having higher top density than the powder 14. Upon heating and compacting at suitable temperatures and pressures the powder 16 with higher top density is compacted to full density whereas the powder 14 is compacted to a density of the order of about 40% of full density to achieve the desired porous surface suitable for attachment to bone by bone growth.

## 0075378

CLAIMS:-

1.    A powder metallurgy prosthesis device for implanting in a human body and having a portion thereof for bonding to a bone, characterised in that said device comprises a first portion of essentially fully dense alloy (16) and a second portion including a fully dense insert (12) with an alloy powder layer (14) bonded thereto with said powder layer (14) being less dense than said essentially fully dense portion.

2.    A powder metallurgy prosthesis device for implanting in a human body and having a portion thereof for bonding to a bone, characterised in that said device comprises a first portion of essentially fully dense alloy powder (16) and a second portion including a fully dense insert (12) with an alloy powder layer (14) bonded thereto with said powder layer being less dense than said essentially fully dense portion.

3.    A device according to claim 1 or claim 2, characterised in that said alloy powder layer (14) has surface pores of a diameter within the range of 100 to 400 micrometers.

4.    A device according to claim 1 or claim 2, characterised in that said alloy powder layer (14) has a powder particle size of -35 to +40 U.S. Standard mesh.

5.    A device according to any one of the preceding claims, characterised in that said first portion is of an alloy powder having a powder particle size -35 U.S. Standard mesh.

6.    A device according to any one of the preceding claims, characterised in that said alloy powder is inert to human body fluids.

7.      A device according to any one of the preceding claims, characterised in that said alloy powder is an alloy consisting of titanium, cobalt base alloy or stainless steel.

8.      A device according to any one of the preceding claims, characterised in that said powder layer (14) is less than 70% dense.

9.      A powder metallurgy method for producing a prosthesis device for implanting in a human body and having a portion thereof for bonding to a bone, characterised in that said method comprises producing a mould (10) having a mould cavity conforming substantially to the configuration of said prosthesis device, filling a first portion of said mould cavity with alloy powder (16), positioning in a second portion of said mould a fully dense insert (12), surrounding said insert (12) with a layer of alloy powder (14) said alloy powder (16) in said first portion being finer than said alloy powder (14) in said second portion, heating said mould (10) to an elevated temperature and while at elevated temperature subjecting said mould (10) to isostatic pressure to compact said powder, with the powder (16) of said first portion being compacted to essentially full density and the powder (14) of said second portion being bonded to said insert (12) and compacted to a density less than fully dense.

10.     A method according to claim 9, characterised in that the alloy powder (16) of said first portion has a powder particle size of -35 U.S. Standard mesh  and the alloy powder (14) of said second portion has a powder particle size of -35 +.60 U.S. Standard mesh.

11.    A method according to claim 9 or 10, characterised in that said alloy powder is inert to human body fluids.

12.    A method according to claim 9,10 or 11 characterised in that said alloy powder is an alloy consisting of titanium, cobalt base alloy or stainless steel.

13.    A method according to any one of claims 9 to 11, characterised in that said powder (14) of said second portion is compacted to a density of less than 70% of full density.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | A 61 F 1/03 |
| X | US-A-3 843 975 (R.TRONZO) *Column 2, lines 1-68* | 1-3,6-8 | A 61 L 17/00 |
| Y | | 9 | |
| A | | 11-13 | |
| | --- | | |
| Y | MECHANICAL ENGINEERING, vol. 94, no. 2, February 1972, page 36; "Porous Prosthesis". *The whole article* | 9 | |
| | --- | | |
| X | US-A-3 605 123 (H.HAHN) *Column 3, lines 30-45; column 3, line 55 - column 4, line 3* | 1-3,6,7 | |
| | --- | | TECHNICAL FIELDS SEARCHED (Int. Cl. ³) |
| A | US-A-4 156 943 (J.COLLIER) *Claims 1-11* | 1-8,10-13 | A 61 F A 61 L |
| | --- | | |
| A | US-A-3 855 638 (R.PILLIAR) *Column 3, line 45 - column 4, line 43* | 1-8,10-13 | |
| | ----- | | |

The present search report has been drawn up for all claims

| Place of search THE HAGUE | Date of completion of the search 19-01-1983 | Examiner LEVENBACH J.H. |
|---|---|---|